# FASCICULE DE BREVET EUROPEEN

(11) **EP 1 495 753 B1**
(45) Date de publication et mention de la délivrance du brevet: **15.08.2007**
(21) Numéro de dépôt: 04291380.6
(22) Date de dépôt: 03.06.2004
(51) Int. Cl.: A61Q 5/00, A61Q 19/00, A61Q 19/08, A61K 8/44

(54) **Utilisation de (2S,3R,4S)-4-hydroxyisoleucine dans des compositions cosmétiques pour application topique.**
Verwendung von (2S,3R,4S)-4-Hydroxyisoleucin in kosmetischen Zubereitungen zur topischen Anwendung.
Use of (2S,3R,4S)-4-hydroxyisoleucine in cosmetic compositions for topical application.

(30) Priorité: 18.06.2003 FR 0307358
(43) Date de publication de la demande: 12.01.2005
(73) Titulaire: CASTER, 75008 Paris (FR); CENTRE NATIONAL DE LA RECHERCHE SCIENTIFIQUE (CNRS), 75794 Paris Cedex 16 (FR)
(72) Inventeur: Potier, Pierre, 75007 Paris (FR); Ouazzani, Jamal, 91300 Massy (FR); Rodelet, Jean-Francois, 92100 Boulogne-Billancourt (FR); Sasaki, Nobumichi André, 91940 Les Ulis (FR); Zhu Wang, Qian, 91190 Gif-sur-Yvette (FR)
(74) Mandataire: Zapalowicz, Francis

(56) Documents cités:
- EP-A- 0 587 476
- FR-A- 2 745 718
- FR-A- 2 776 184
- FR-A- 2 797 767

## Description

La présente invention concerne l'utilisation de la (2S,3R,4S)-4-hydroxyisoleucine en application topique pour lutter contre les effets du vieillissement de la peau et du cuir chevelu.

La (2S,3R,4S)-4-hydroxyisoleucine est un acide aminé rare, absent chez les humains et les animaux, que l'on ne trouve que dans certaines espèces végétales. Cet acide aminé est connu depuis plusieurs années pour son effet insulino-régulateur et est utilisé en tant que principe actif dans le traitement du diabète.

La demanderesse a découvert que cette même molécule, administrée non pas par voie interne mais par application topique, permet de combattre les signes naturels du vieillissement de la peau et du cuir chevelu et en particulier l'alopécie androgéno-génétique, ou alopécie de type masculin.

La présente invention a par conséquent pour objet des compositions cosmétiques à application topique contenant, dans un support cosmétiquement acceptable, de 0,02 à 2% en poids de (2S,3R,4S)-4-hydroxyisoleucine.

Elle a également pour objet l'utilisation de la (2S,3R,4S)-4-hydroxyisoleucine pour combattre les signes naturels du vieillissement de la peau et du cuir chevelu et notamment la chute des cheveux liée au vieillissement.

Elle a en outre pour objet un procédé de traitement cosmétique de la peau et du cuir chevelu comprenant l'application sur la peau ou le cuir chevelu d'une composition cosmétique selon l'invention pendant une durée et à une fréquence suffisante pour l'obtention de l'effet cosmétique recherché.

Comme indiqué ci-dessus, la (2S,3R,4S)-4-hydroxyisoleucine de formule CH₃-CHOH-CHCH₃-CHNH₂-COOH est un acide aminé naturel très rare que l'on ne rencontre que dans certaines espèces végétales parmi lesquelles on peut citer *Dioscorea deltoidea, Balanires aegyptiaca, Trigonella* *foenum-graecum* (fénugrec) et *Solanum laciniatum*. Une méthode de synthèse de cette molécule a été décrite dans la demande de brevet internationale WO 01/72688.

La demanderesse, dans le cadre d'un programme de recherches étudiant l'action physiologique de la 4-hydroxyisoleucine, a découvert avec surprise que cette molécule provoquait, au niveau cellulaire, une modification du taux d'expression de certains gènes.

Les résultats de ces recherches, obtenus par la méthode « cDNA-macroarrays » et confirmés par PCR quantitative après transcription inverse des ARN messagers de cellules épidermiques (méthode RT-PCR quantitative), révèlent en particulier :
- une sous-expression significative de la *Cellular Retinoic Acid Binding Protein* (CRAB-P-2) : cette protéine a un rôle régulateur de la quantité d'acide rétinoïque libre et sa sous-expression se traduit par une augmentation de la disponibilité de l'acide rétinoïque au niveau cellulaire ;
- une sur-expression significative du *Bullous Pemphigoïd Antigen 1* (BPAG-1) : cette protéine intervient dans la consolidation du cyctosquelette et dans la cohésion cellulaire ;
- une surexpression significative du récepteur de JL1-R1, ainsi que la diminution de l'expression des récepteurs de TNS & CRAB-2 contribuent à la préservation de l'équilibre de l'effet pro-inflammatoire et anti-inflammatoire qui se traduit globalement par une diminution de l'inflammation.

Or, on connaît d'une part le rôle de l'acide rétinoïque dans le renouvellement cellulaire, et en particulier dans le renouvellement des cellules de l'épiderme, et, d'autre part, l'implication des phénomènes inflammatoires dans la dégradation de la matrice extracellulaire. De l'ensemble de ces effets biologiques, à savoir la stimulation du renouvellement des cellules épidermiques, la prévention de la dégradation de la matrice extracellulaire et le renforcement du cytosquelette et de la cohésion cellulaire, la demanderesse a déduit que la (2S,3R,4S)-4-hydroxyisoleucine devait avoir un effet anti-vieillissement au niveau de l'épiderme.

Les compositions cosmétiques de la présente invention peuvent se présenter sous n'importe quelle forme permettant une application topique aisée, par exemple sous forme d'une lotion, d'un sérum, d'une crème, d'un lait ou d'une pommade.

La concentration de (2S,3R,4S)-4-hydroxyisoleucine dans ces compositions dépend d'un certain nombre de paramètres tels que la nature ou la gravité du trouble cosmétique à traiter (rides, tâches de vieillissement, chute des cheveux), la fréquence et la durée d'application et la présence éventuelle d'autres principes actifs cosmétiques.

La demanderesse a constaté que des résultats satisfaisants étaient obtenus avec des concentrations en (2S,3R,4S)-4-hydroxyisoleucine comprises entre 0,02 % et 2 % en poids rapportées au poids total de la composition cosmétique.

L'effet anti-vieillissement au niveau de l'épiderme a été confirmé *in vivo* dans une étude portant sur les propriétés anti-chute des cheveux de la (2S,3R,4S)-4-hydroxyisoleucine, réalisée sur des sujets volontaires atteints d'alopécie présentant une proportion de cheveux en phase télogène supérieure ou égale à 15 %. Cette étude, dont les conditions sont décrites en détail dans l'exemple de réalisation ci-après, a montré que la (2S,3R,4S)-4-hydroxyisoleucine était particulièrement efficace lorsqu'elle est utilisée en tant que principe actif dans une composition anti-chute des cheveux contenant déjà une association particulière de principes actifs anti-chute. Cette composition anti-chute est décrite dans la demande FR 2 776 184 dont la demanderesse est titulaire. Elle contient un extrait aqueux du champignon *Lentinus Edodes* riche en 1,3-β-D-1,6-β-D-glucane, des oligomères procyanidoliques extraits de la cuticule du pépin de raisin, et de l'huile essentielle extraite des fleurs de *Cananga Odorata.*

Par conséquent, dans un mode de réalisation préféré de l'invention, la composition cosmétique à application topique de la présente invention est une composition capillaire anti-chute des cheveux et en particulier une composition anti-chute contenant, en plus de 0,02 à 2% en poids de (2S,3R,4S)-4-hydroxyisoleucine, un extrait aqueux du champignon *Lentinus Edodes* riche en 1,3-β-D-1,6-β-D-glucane, des oligomères procyanidoliques extraits de la cuticule du pépin de raisin, et de l'huile essentielle extraite des fleurs de *Cananga Odorata.*

L'obtention de l'effet cosmétique recherché nécessite l'application répétée du principe actif, sous forme d'une composition cosmétique telle que décrite ci-avant, pendant une durée et à une fréquence suffisantes qui peuvent facilement être déterminées par l'homme du métier grâce à ses connaissances techniques générales. Pour l'obtention d'un effet anti-chute des cheveux, la composition cosmétique selon l'invention est appliquée de préférence pendant une durée d'au moins 12 semaines, en particulier d'au moins 16 semaines, à une fréquence de préférence au moins égale à 1 application toutes les 72 heures, en particulier au moins égale à 1 application toutes les 48 heures.

### Exemple 1

On a testé l'efficacité anti-chute des cheveux de la (2S,3R,4S)-4-hydroxyisoleucine en l'incorporant en une concentration de 0,05 % en poids dans une composition capillaire antichute du commerce (Phytoaxil®) contenant un extrait aqueux du champignon *Lentinus Edodes* riche en 1,3-β-D-1,6-β-D-glucane, des oligomères procyanidoliques extraits de la cuticule du pépin de raisin, et de l'huile essentielle extraite des fleurs de *Cananga Odorata,* et en comparant les résultats obtenus avec cette nouvelle composition à ceux obtenus avec la composition Phytoaxil® seule.

Cette étude a été réalisée sur un échantillon de 25 volontaires pour chaque composition testée. Seuls ont été inclus dans l'étude des hommes âgés de 20 à 50 ans (âge moyen de chaque groupe 37 ans) présentant une alopécie, c'est-à-dire présentant une proportion de cheveux en phase télogène, déterminée par vidéotrichogramme, supérieure à 15 %.

L'appareil utilisé est un vidéomicroscope muni d'un objectif x 25, mobile et à fibre optique, couplé à un système informatique d'acquisition d'images. L'objectif est mis directement sur la zone étudiée, l'image est numérisée, enregistrée et analysée à l'aide d'un logiciel spécifique (COUNT-HAIR®) mis au point par la société DERMSCAN qui permet de compter les cheveux et de mesurer leur longueur. Les paramètres évalués sont
a) la densité capillaire,
b) le nombre et la proportion de cheveux en phase anagène (phase de croissance),
c) le nombre et la proportion de cheveux en phase télogène (arrêt de croissance) et
d) le diamètre moyen des cheveux.

Le déroulement chronologique de l'étude est le suivant :
semaine 0, jour 0 : rasage des cheveux dans une zone déterminée et enregistrement du premier vidéotrichogramme ;
semaine 0, jour 2 : deuxième vidéotrichogramme, détermination de la proportion de cheveux en phase télogène et sélection des volontaires chez lesquels cette proportion est supérieure à 15 %, distribution de la composition à tester (Phytoaxil® ou Phytoaxil® + 0,05 % de (2S,3R,4S)-4-hydroxyisoleucine) en une quantité suffisante pour quatre semaines de traitement à raison d'une application tous les deux jours ;
semaine 4, jour 0 : rasage de la zone de cheveux, distribution de la composition à tester en une quantité suffisante pour quatre semaines de traitement à raison d'une application tous les deux jours ;
semaine 8, jour 0 : rasage de la zone de cheveux, vidéotrichogramme ;
semaine 8, jour 2 : vidéotrichogramme, distribution de la composition à tester en une quantité suffisante pour quatre semaines de traitement à raison d'une application tous les deux jours ;
semaine 12, jour 0 : rasage de la zone de cheveux, distribution de la composition à tester en une quantité suffisante pour quatre semaines de traitement à raison d'une application tous les deux jours ;
semaine 16, jour 0 : rasage de la zone de cheveux, vidéotrichogramme,
semaine 16, iour 2 : vidéotrichogramme.

A la fin de l'étude, on dispose ainsi de la proportion initiale de cheveux en phase télogène et de la proportion de cheveux en phase télogène au bout de 8 et 16 semaines.

L'exploitation statistiques des résultats ainsi obtenus pour les deux groupes de sujets volontaires se fait selon le test de Student sur données appariées, en calculant la probabilité p d'observer un écart entre la proportion de cheveux en phase télogène avant et après traitement au moins aussi grand que celui qui a réellement été observé, si l'hypothèse nulle était vraie, c'est-à-dire si le traitement n'avait aucun effet.

Si la valeur de p calculée à partir des résultats obtenus est inférieure à 5 % (0,05), on rejette l'hypothèse nulle et on conclut à une différence significative due au traitement.

Le tableau 1 ci-dessous montre la valeur de p calculée à partir des résultats des mesures du diamètre capillaire, de la proportion de cheveux en phase télogène et de la densité capillaire réalisées sur les volontaires des deux groupes au bout de 8 semaines de traitement et de 16 semaines de traitement.

**Tableau 1**

| Valeurs de p (Test de Student) | | |
|---|---|---|
| | Phytoaxil® + 4-hydroxyisoleucine | témoin |
| Diamètre des cheveux après 8 semaines | 0,260 | 0,070 |
| Diamètre des cheveux après 16 semaines | **0,004** | 0,784 |
| Phase télogène après 8 semaines | 0,097 | 0,400 |
| Phase télogène après 16 semaines | **0,004** | 0,862 |
| Densité capillaire après 8 semaines | 0,078 | 0,711 |
| Densité capillaire après 16 semaines | **0,001** | 0,115 |

Ces résultats montrent clairement qu'au bout de 16 semaines de traitement avec la composition selon l'invention contenant 0,05 % en poids de (2S,3R,4S)-4-hydroxyisoleucine, on observe une augmentation significative de la densité capillaire et du diamètre des cheveux, et une réduction significative du nombre de cheveux en phase télogène.

### Exemple 2

| Lotion capillaire anti-chute | |
|---|---|
| (2S,3R,4S)-4-hydroxyisoleucine | 0,05 % |
| Glycérine | 9,00 % |
| Diméthylisosorbide | 6,00 % |
| Parfum | 4,50 % |
| Extrait de *Solanum Tuberosum* | 1,00 % |
| Huile essentielle de *Cananga Odorata* | 1,00 % |
| Extrait de *Serenoa Repens* | 0,20 % |
| Extrait de *Lentinus Edodes* | 0,20 % |
| OPC de raisin | 0,10 % |
| Conservateurs | q.s. |
| Ethanol | 25,00 % |
| Eau distillée | q.s.p. 100 % |

### Exemple 3

| Sérum capillaire anti-chute | |
|---|---|
| (2S,3R,4S)-4-hydroxyisoleucine | 0,05 % |
| Ethanol (96 % vol.) | 20,00 % |
| Glycérine | 5,00 % |
| Hydrolysat de protéines de soja | 0,80 % |
| Extrait huileux de *Prunus africana* | 0,30 % |
| PPG-1-PEG-9 Lauryl Glycol Ether | 5,00 % |
| Parfum | q.s. |
| Conservateur (éthylparaben, butylparaben, phénoxyéthanol) | q.s. |
| Eau déminéralisée | q.s.p.100 % |

### Exemple 4

| Sérum anti-rides pour la peau | |
|---|---|
| 4-hydroxyisoleucine | 2,00 % |
| PEG-8 caprylic/capric glycérides | 30,00 % |
| Polyglycéryl-3 Diisostéarate | 20,00 % |
| Octadécyl myristate | 12,00 % |
| Palmitate d'ascorbyle | 1,00 |
| Extrait d'écorce de *Pinus pinaster* | 0,20 % |
| Parfum | q.s. |
| Conservateur (éthylparaben, butylparaben, phénoxyéthanol) | q.s. |
| Eau déminéralisée | q.s.p.100 % |

### Exemple 5 (nom conforme à l'invention)

| Crème de beauté anti-rides | |
|---|---|
| (2S,3R,4S)-4-hydroxyisoleucine | 10,00 % |
| Glycérine | 10,00 % |
| Stéarate de triéthanolamine | 10,00 % |
| Alcool cétylique | 0,50 % |
| Stéarine triple pression | 0,50 % |
| Huile d'amande douce | 5,00 % |
| Vaseline | 5,00 % |
| Diméthicone | 2,00 % |
| Conservateur (parabens) | q.s. |
| Parfum | q.s. |
| Eau déminéralisée | q.s.p. 100 % |

### Exemple 6

| Crème de beauté anti-taches | |
|---|---|
| (2S,3R,4S)-4-hydroxyisoleucine | 0,80 % |
| PEG-6 Stéarate/PEG-32 Stéarate | 6,50 % |
| Cire d'abeille auto-émulsionnable | 4,50 % |
| Huile de vaseline fluide | 4,00 % |
| Diméthicone 200/100 | 3,00 % |
| PEG-30 Castor Oil | 1,50% |
| Diéthyl Trioxopimélate | 0,80 % |
| Parfum | q.s. |
| Conservateur (parabens) | q.s. |
| Eau déminéralisée | q.s.p.100 % |

### Exemple 7

| Lait pour le corps | |
|---|---|
| (2S,3R,4S)-4-hydroxyisoleucine | 0,03 % |
| Huile de vaseline fluide | 8,00 % |
| Huile d'amande douce | 4,00 % |
| Stéarate de glycérol auto-émulsionnable | 3,50 % |
| Alcool cétylique | 2,00 % |
| Alcool stéarylique oxyéthyléné (20 OE) | 1,50 % |
| Diméthicone 200/100 | 1,00 % |
| Carbomer | 0,30 % |
| Menthol | 0,10 % |
| Parfum | q.s. |
| Conservateur (parabens) | 0,30 % |
| Eau déminéralisée | q.s.p.100 % |

## Revendications

1. Composition cosmétique à application topique contenant, dans un support cosmétiquement acceptable, de 0,02 à 2 % en poids de (2S,3R,4S)-4-hydroxyisoleucine.

2. Composition cosmétique à application topique selon la revendication 1 **caractérisée par le fait qu'**elle se présente sous forme d'une lotion, d'un sérum, d'une crème, d'un lait ou d'une pommade.

3. Composition cosmétique à application topique selon la revendication 1 ou 2 **caractérisée par le fait qu**'il s'agit d'une composition capillaire anti-chute.

4. Composition cosmétique à application topique selon la revendication 3, **caractérisée par le fait qu**'elle comprend en outre
a) un extrait aqueux du champignon *Lentinus Edodes* riche en 1,3-β-D-1,6-β-D-glucane,
b) des oligomères procyanidoliques extraits de la cuticule du pépin de raisin, et
c) de l'huile essentielle extraite des fleurs de *Cananga Odorata.*

5. Composition cosmétique à application topique selon la revendication 1 ou 2, **caractérisée par le fait qu**'il s'agit d'une crème antirides et/ou d'une crème contre les tâches de vieillissement.

6. Utilisation de (2S,3R,4S)-4-hydroxyisoleucine en application topique pour combattre les signes naturels du vieillissement de la peau et du cuir chevelu.

7. Utilisation selon la revendication 6 pour combattre la chute des cheveux liée au vieillissement.

8. Utilisation selon la revendication 7, **caractérisée par le fait que** l'on utilise la (2S,3R,4S)-4-hydroxyisoleucine en association avec un ou plusieurs autres principes actifs anti-chute.

9. Utilisation selon la revendication 8, **caractérisée par le fait que** l'on utilise la (2S,3R,4S)-4-hydroxyisoleucine en association avec
e) un extrait aqueux du champignon *Lentinus Edodes* riche en 1,3-β-D-1,6-β-D-glucane,
f) des oligomères procyanidoliques extraits de la cuticule du pépin de raisin, et
g) de l'huile essentielle extraite des fleurs de *Cananga Odorata.*

10. Procédé de traitement cosmétique de la peau et/ou du cuir chevelu comprenant l'application sur la peau et/ou le cuir chevelu d'une composition cosmétique selon l'une quelconque des revendications 1 à 5, pendant une durée et à une fréquence suffisante pour l'obtention de l'effet cosmétique recherché.

11. Procédé de traitement cosmétique du cuir chevelu comprenant l'application sur le cuir chevelu d'une composition cosmétique selon l'une des revendications 1 à 4 pendant une durée d'au moins 12 semaines, de préférence d'au moins 16 semaines, à une fréquence au moins égale à 1 application/72 heures, de préférence au moins égale à 1 application/48 heures.

## Claims

1. Cosmetic composition for topical application containing, in a cosmetically acceptable carrier, from 0.02 to 2% by weight of (2S,3R,4S)-4-hydroxyisoleucine.

2. Cosmetic composition for topical application according to Claim 1, **characterized in that** it is provided in the form of a lotion, a serum, a cream, a milk or an ointment.

3. Cosmetic composition for topical application according to Claim 1 or 2, **characterized in that** it is an anti-hair loss hair composition.

4. Cosmetic composition for topical application according to Claim 3, **characterized in that** it additionally comprises
a) an aqueous extract of the mushroom *Lentinus edodes* rich in 1,3-β-D-1,6-β-D-glucan,
b) procyanidolic oligomers extracted from grapeseed cuticle, and
c) essential oil extracted from the flowers of *Cananga odorata.*

5. Cosmetic composition for topical application according to Claim 1 or 2, **characterized in that** it is an anti-wrinkle cream and/or a cream against ageing spots.

6. Use of (2S,3R,4S)-4-hydroxyisoleucine in topical application for combating the natural signs of ageing of the skin and of the scalp.

7. Use according to Claim 6, for combating hair loss linked to ageing.

8. Use according to Claim 7, **characterized in that** (2S,3R,4S)-4-hydroxyisoleucine is used in combination with one or more other anti-hair loss active ingredients.

9. Use according to Claim 8, **characterized in that** (2S,3R,4S)-4-hydroxyisoleucine is used in combination with
e) an aqueous extract of the mushroom *Lentinus edodes* rich in 1,3-β-D-1,6-β-D-glucan
f) procyanidolic oligomers extracted from grapeseed cuticle, and
g) essential oil extracted from the flowers of *Cananga odorata.*

10. Method for the cosmetic treatment of the skin and/or of the scalp comprising the application to the skin and/or to the scalp of a cosmetic composition according to any one of Claims 1 to 5, for a sufficient duration and at a sufficient frequency for the desired cosmetic effect to be obtained.

11. Method for the cosmetic treatment of the scalp comprising the application to the scalp of a cosmetic composition according to one of Claims 1 to 4 for a period of at least 12 weeks, preferably of at least 16 weeks, at a frequency at least equal to 1 application/72 hours, preferably at least equal to 1 application/48 hours.

## Patentansprüche

1. Kosmetische Zusammensetzung für den topischen Auftrag, die in einem kosmetisch akzeptablen Träger 0,02 bis 2 Gew.-% (2S,3R,4S)-4-Hydroxyisoleucin enthält.

2. Kosmetische Zusammensetzung für den topischen Auftrag nach Anspruch 1, **dadurch gekennzeichnet, dass** sie in Form einer Lotion, eines Serums, einer Creme, einer Milch oder einer Salbe vorliegt.

3. Kosmetische Zusammensetzung für den topischen Auftrag nach Anspruch 1 oder 2, **dadurch gekennzeichnet, dass** es sich dabei um eine Zusammensetzung gegen Haarausfall handelt.

4. Kosmetische Zusammensetzung für den topischen Auftrag nach Anspruch 3, **dadurch gekennzeichnet, dass** sie außerdem umfasst
a) einen wässrigen Extrakt des Pilzes Lentinus Edodes, der reich an 1,3-β-D-1,6-β-D-glucan ist,
b) Procyanidol-Oligomere, die aus der Kutikula des Traubenkerns extrahiert worden sind, und
c) ein essentielles Öl, das aus Blüten von Cananga Odorata extrahiert worden ist.

5. Kosmetische Zusammensetzung für den topischen Auftrag nach Anspruch 1 oder 2, **dadurch gekennzeichnet, dass** es sich dabei um eine Antifalten-Creme und/oder eine Creme gegen die Bildung von Altersflecken handelt.

6. Verwendung von (2S,3R,4S)-4-Hydroxyisoleucin beim topischen Auftrag zur Bekämpfung von natürlichen Anzeichen des Alterns der Haut und der Kopfhaut.

7. Verwendung nach Anspruch 6 zur Bekämpfung von Haarausfall, der im Zusammenhang mit dem Altern steht.

8. Verwendung nach Anspruch 7, **dadurch gekennzeichnet, dass** man das (2S,3R,4S)-4-Hydroxyisoleucin in Kombination mit einem oder mehreren Wirkstoffen gegen Haarausfall verwendet.

9. Verwendung nach Anspruch 8, **dadurch gekennzeichnet, dass** man das (2S,3R,4S)-4-Hydroxyisoleucin verwendet in Kombination mit
e) einem wässrigen Extrakt des Pilzes Lentinus Edodes, der reich an 1,3-β-D-1,6-β-D-glucan, ist,
f) Procyanidol-Oligomeren, die aus der Kutikula des Traubenkerns extrahiert worden sind, und
g) dem essentiellen Öl, das aus Blüten von Cananga Odorata extrahiert worden ist.

10. Verfahren zur kosmetischen Behandlung der Haut und/oder der Kopfhaut, das umfasst das Aufbringen einer kosmetischen Zusammensetzung nach einem der Ansprüche 1 bis 5 auf die Haut und/oder die Kopfhaut für eine Zeitdauer und mit einer Häufigkeit, die ausreichen, um den gewünschten kosmetischen Effekt zu erzielen.

11. Verfahren zur kosmetischen Behandlung der Kopfhaut, das umfasst das Aufbringen einer kosmetischen Zusammensetzung nach einem der Ansprüche 1 bis 4 auf die Kopfhaut für eine Zeitdauer von mindestens 12 Wochen, vorzugsweise von mindestens 16 Wochen, mit einer Häufigkeit von mindestens einem Auftrag pro 72 h, vorzugsweise von mindestens einem Auftrag pro 48 h.
